(19) Europäisches Patentamt
European Patent Office
Office européen des brevets

(11) EP 4 434 527 A1

(12) EUROPEAN PATENT APPLICATION
published in accordance with Art. 153(4) EPC

(43) Date of publication:
**25.09.2024 Bulletin 2024/39**

(21) Application number: **22896089.4**

(22) Date of filing: **17.11.2022**

(51) International Patent Classification (IPC):
***A61K 31/704*** *(2006.01)* ***A61K 31/454*** *(2006.01)*
***A61P 35/00*** *(2006.01)*

(52) Cooperative Patent Classification (CPC):
**A61K 31/454; A61K 31/704; A61P 35/00**

(86) International application number:
**PCT/KR2022/018180**

(87) International publication number:
**WO 2023/090888 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.11.2021 KR 20210160623**

(71) Applicants:
- **KBlueBio Inc**
**Jeollanam-do 58128 (KR)**
- **Industry Foundation of Chonnam National University**
**Gwangju 61186 (KR)**

(72) Inventors:
- **KIM, Hye Ran**
**Gwangju 61698 (KR)**
- **LEE, Young Eun**
**Gwangju 61742 (KR)**
- **SHIN, Myung Guen**
**Gwangju 61143 (KR)**

(74) Representative: **Synergy IP Group AG**
**Leonhardsgraben 52**
**Postfach**
**4001 Basel (CH)**

(54) **COMPOSITION FOR PREVENTION OR TREATMENT OF MULTIPLE MYELOMA COMPRISING NOVEL TETRACYCLIC TRITERPENE COMPOUND AS ACTIVE INGREDIENT**

(57) The present invention provides a novel multi-fused-ring compound and a composition for preventing or treating multiple myeloma comprising the same as an active ingredient. The compound of the present invention is a derivative synthesized from the natural compound ginsenoside as a starting material, and shows no cytotoxicity to normal hematopoietic stem cells while exhibiting a significant killing effect on various multiple myeloma cell lines, indicating that it may be administered for a long period of time without side effects. In addition, the compound of the present invention exhibits a significant synergistic effect when co-administered with the immunomodulator thalidomide or its analog lenalidomide, and thus may be useful as an efficient therapeutic agent or therapeutic aid agent composition for multiple myeloma, an incurable disease.

EP 4 434 527 A1

FIG. 1

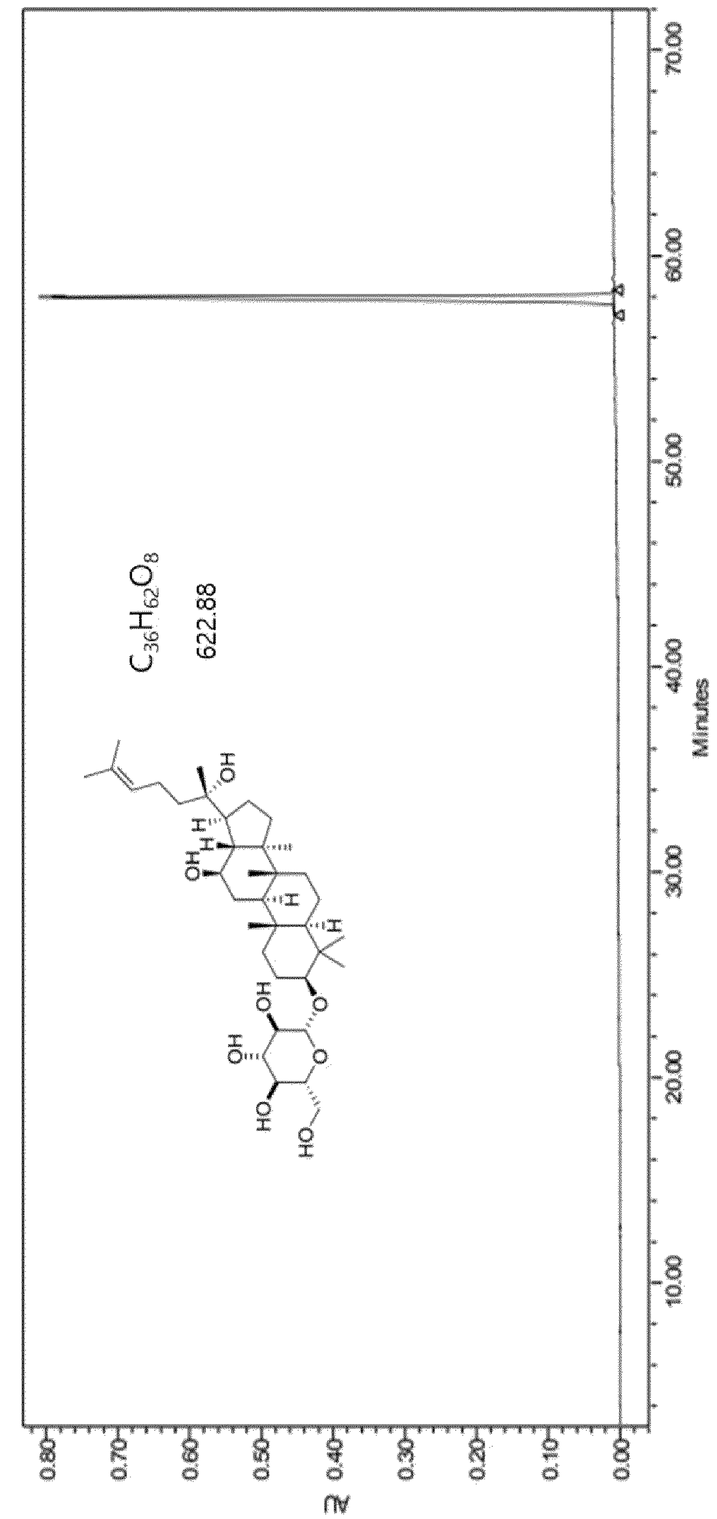
AU
0.80
0.70
0.60
0.50
0.40
0.30
0.20
0.10
0.00
10.00
20.00
30.00
40.00
50.00
60.00
70.00
Minutes
$C_{36}H_{62}O_8$
622.88

## Description

### Technical Field

The present invention relates to a novel compound having a four-fused-ring structure and a composition comprising the same for preventing or treating bone marrow cancer, including multiple myeloma.

### Background Art

Among blood cancers that occur in adults, the incidence of multiple myeloma has increased most rapidly with population aging. Treatment of multiple myeloma has been attempted based on melphalan and prednisone therapy, but anticancer drugs based on the immunomodulators thalidomide and lenalidomide and the proteasome inhibitor bortezomib are widely being used in clinical practice. However, these drugs cause pancytopenia by destroying normal hematopoietic stem cells or inhibiting their function, and also cause serious side effects such as the occurrence of peripheral neuritis, embolism/thrombosis, and secondary primary cancer. In addition, as the drug resistance of multiple myeloma increases, mortality due to multiple myeloma is also significantly increasing.

Accordingly, in order to successfully treat multiple myeloma and to improve patient survival rates, the development of new anticancer drugs with dramatically reduced side effects mentioned above is required. Therefore, the present invention is intended to provide an efficient therapeutic composition for multiple myeloma, which is an incurable blood cancer, by discovering a novel small-molecule compound that exhibits a significant killing effect on multiple myeloma cells, verifying the anticancer effect of the compound in various ways through cell line and animal experiments, and observing that the compound shows no significant toxicity to a group of peripheral blood hematopoietic stem cells derived from a normal donor.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made extensive research efforts to develop an effective and side-effect-free small-molecule compound for the treatment of incurable blood cancers caused by abnormal proliferation of plasma cells, including multiple myeloma. As a result, the present inventors have found that a tetracyclic triterpene compound represented by Formula (1) below exhibits a significant killing effect on various multiple myeloma cell lines without showing significant toxicity to normal hematopoietic stem cells, and exhibits a therapeutic effect better than a commercially available drug *in vivo,* thereby completing the present invention.

Therefore, an object of the present invention is to provide a novel tetracyclic triterpene compound represented by Formula (1) below and a composition for preventing or treating multiple myeloma comprising the same as an active ingredient.

Other objects and advantages of the present invention will be more apparent from the following detailed description, the appended claims, and the accompanying drawings.

### Technical Solution

According to one aspect of the present invention, the present invention provides a composition for preventing or treating cancer comprising a compound represented by the following Formula 1 or a pharmaceutically acceptable salt thereof as an active ingredient:

**Formula 1**

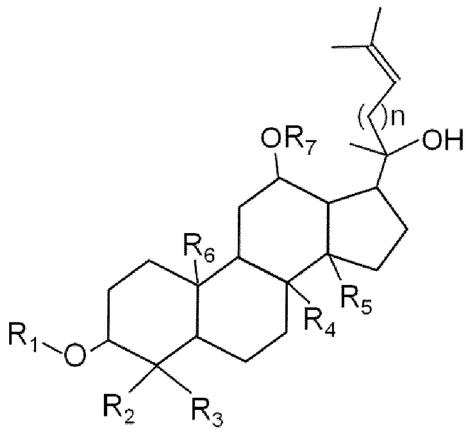

In the Formula, $R_1$ is

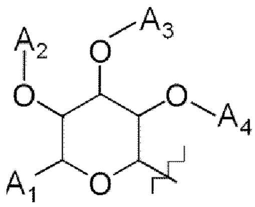

(where $A_1$ is $C_1$-$C_3$ alkyl unsubstituted or substituted with hydroxy, and $A_2$ to $A_4$ are each independently hydrogen or $C_1$-$C_3$ alkyl); $R_2$ to $R_7$ are each independently hydrogen or $C_1$-$C_3$ alkyl; and n is an integer ranging from 1 to 3.

The present inventors have made extensive research efforts to develop an effective and side-effect-free small-molecule compound for the treatment of incurable blood cancers caused by abnormal proliferation of plasma cells, including multiple myeloma. As a result, the present inventors have found that the compound represented by Formula (1) exhibits a significant killing effect on various multiple myeloma cell lines without showing significant toxicity to normal hematopoietic stem cells, and exhibits a therapeutic effect better than a commercially available drug *in vivo.*

In the present specification, the term "alkyl" refers to a straight-chain or branched saturated hydrocarbon group, and includes, for example, methyl, ethyl, propyl, isopropyl, etc. The term "$C_1$-$C_3$ alkyl" refers to an alkyl group having an alkyl unit having 1 to 3 carbon atoms, and when the $C_1$-$C_3$ alkyl is substituted, the carbon atom number of the substituent is not included.

According to a specific embodiment of the present invention, $A_1$ is hydroxymethyl, and $A_2$ to $A_4$ are each hydrogen.

According to a specific embodiment of the present invention, $R_2$ to $R_6$ are each independently hydrogen or methyl.

According to a specific embodiment of the present invention, n is 2.

More specifically, the compound represented by Formula 1 is a compound represented by the following Formula 2:

**Formula 2**

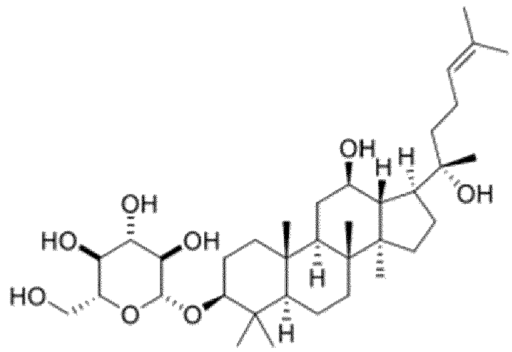

In the present specification, the term "pharmaceutically acceptable salt" includes a salt derived from a pharmaceutically acceptable inorganic acid, organic acid, or base. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, trifluroacetic acid, citric acid, methanesulfonic acid, formic acid, benzoic acid, malonic acid, naphthalene-2-sulfonic acid, benzenesulfonic acid, and the like. Salts derived from suitable bases include salts of alkali metals such as sodium, alkaline earth metals such as magnesium, ammonium, and the like.

In the present specification, the term "prevention" means inhibiting the occurrence of a disorder or a disease in a subject who has never been diagnosed as having the disorder or disease, but is likely to suffer from such disorder or disease.

In the present specification, the term "treatment" means (a) inhibiting the progress of a disorder, disease or symptom; (b) alleviating the disorder, disease or symptom; or (c) eliminating the disorder, disease or symptom. The composition of the present invention functions to inhibit the proliferation of tumor cell lines, induce apoptosis, and reduce the tumor lesion area, thereby inhibiting the progress of symptoms caused by tumors, or eliminating or alleviating the symptoms. Thus, the composition of the present invention may serve as a therapeutic composition for the disease alone, or may be administered in combination with other pharmacological ingredients having anticancer effects and applied as a therapeutic aid for the disease. Accordingly, as used in the present specification, the term "treatment" or "therapeutic agent" encompasses the meaning of "treatment aid" or "therapeutic aid agent".

In the present specification, the term "administration" or "administering" means administering a therapeutically effective amount of the composition of the present invention directly to a subject so that the same amount is formed in the subject's body.

In the present specification, the term "therapeutically effective amount" refers to an amount of the composition sufficient to provide a therapeutic or prophylactic effect to a subject to whom/which the composition of the present invention is to be administered. Accordingly, the term "therapeutically effective amount" is meant to include a "prophylactically effective amount".

In the present specification, the term "subject" includes, without limitation, humans, mice, rats, guinea pigs, dogs, cats, horses, cows, pigs, monkeys, chimpanzees, baboons or rhesus monkeys. Specifically, the subject of the present invention is a human.

According to a specific embodiment of the present invention, the cancer to be prevented or treated by the composition of the present invention is blood cancer.

In the present specification, the term "blood cancer" refers to a malignant tumor that occurs in white blood cells, red blood cells, and platelets, which are components of blood; bone marrow where blood is produced; or the lymphatic system including lymphocytes, lymph nodes, and lymphatic vessels, which make up the immune system.

More specifically, the blood cancer to be prevented or treated by the composition of the present invention is bone marrow cancer. Most specifically, the blood cancer is multiple myeloma.

In the present specification, the term "multiple myeloma" refers to blood cancer that is caused by abnormal differentiation and proliferation of plasma cells, which is a type of white blood cell responsible for the immune system, in the bone marrow.

According to a specific embodiment of the present invention, the composition of the present invention further comprises a compound represented by the following Formula 3 or a pharmaceutically acceptable salt thereof:

**Formula 3**

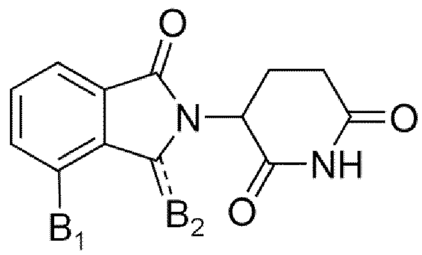

In the Formula, $B_1$ is hydrogen or $-NH_2$, - - - represents a single bond or double bond, and $B_2$ is hydrogen when - - - represents a single bond, or oxygen when - - - represents a double bond.

More specifically, $B_1$ is $-NH_2$, and $B_2$ is hydrogen. According to the octet rule, it is obvious that when $B_2$ is hydrogen, - - - represents a single bond. The compound of Formula 3, wherein $B_1$ is $-NH_2$ and $B_2$ is hydrogen, is lenalidomide ($C_{13}H_{13}N_3O_3$; 3-(4-amino-1-oxoisoindolin-2-yl)piperidine-2,6-dione).

Lenalidomide is an immunomodulator widely used in the treatment of multiple myeloma and myelodysplastic syndrome, and has anti-angiogenic and anti-inflammatory activities. According to the present invention, the compound of the present invention not only exhibited a therapeutic effect equal to or higher than that of lenalidomide, a representative commercially available therapeutic agent for multiple myeloma, but also exhibited a significant synergistic effect when co-administered with lenalidomide. Accordingly, the compound of the present invention may not only be applied as an anticancer agent alone, but may also be useful as an efficient therapeutic aid agent that is co-administered with lenalidomide or similar immunomodulators.

According to another specific embodiment of the present invention, $B_1$ is hydrogen and $B_2$ is oxygen. According to the octet rule, it is obvious that when $B_2$ is oxygen, - - - represents a double bond. The compound of Formula 3, wherein $B_1$ is hydrogen and $B_2$ is oxygen, is thalidomide ($C_{13}H_{10}N_2O_4$; 2-(2,6-dioxopiperidin-3-yl)-1H-isoindole-1,3 (2H)-dione).

Thalidomide is an analogue, from which lenalidomide is derived, and has been used for a long time in the treatment of multiple myeloma until lenalidomide was approved in the United States in 2005.

When the composition of the present invention is prepared as a pharmaceutical composition, the pharmaceutical composition of the present invention comprises a pharmaceutically acceptable carrier. Examples of the pharmaceutically acceptable carrier that is comprised in the pharmaceutical composition of the present invention include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, which are commonly used in formulation. The pharmaceutical composition of the present invention may further comprise a lubricant, a wetting agent, a sweetening agent, a flavoring agent, an emulsifying agent, a suspending agent, a preservative, and the like, in addition to the above-described ingredients. Suitable pharmaceutically acceptable carriers and agents are described in detail in Remington's Pharmaceutical Sciences (19th ed., 1995).

The pharmaceutical composition of the present invention may be administered via various routes. Specifically, it may be administered parenterally. More specifically, it may be administered orally, intravenously, intra-arterially, subcutaneously, intraperitoneally, intradermally, intramuscularly, intracerebroventricularly, intrathecally, by inhalation, intranasally, intra-articularly, or topically.

An appropriate dosage of the pharmaceutical composition of the present invention may vary depending on various factors such as formulation method, administration mode, patient's age, weight, sex, pathological condition, diet, administration time, administration route, excretion rate, and reaction sensitivity. A preferred dosage of the pharmaceutical composition of the present invention is within the range of 0.0001 to 100 mg/kg for an adult.

The pharmaceutical composition of the present invention may be prepared in a unit dose form or prepared to be contained in a multi-dose container by formulating with a pharmaceutically acceptable carrier and/or excipient, according to a method that may be easily carried out by a person skilled in the art. In this case, the formulation of the pharmaceutical composition may be a solution, suspension, syrup or emulsion in oil or aqueous medium, or an extract, powder, granule, tablet or capsule, and may further comprise a dispersing agent or a stabilizer.

According to another aspect of the present invention, the present invention provides an anticancer composition for co-administration with a compound represented by the following Formula 3 or a pharmaceutically acceptable salt thereof, comprising the above-described compound of Formula 1 or pharmaceutically acceptable salt thereof according to the present invention as an active ingredient:

**Formula 3**

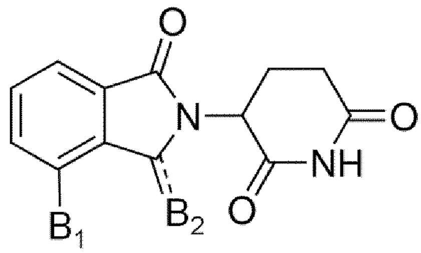

The compounds of Formula 1 and Formula 3 used in the present invention and the type of cancer that can be prevented or treated by these compounds have already been described in detail above, and thus the description thereof will be omitted to avoid excessive overlapping.

As described above, the compound of the present invention exhibited a significant synergistic effect when co-administered with lenalidomide. Thus, the compound of the present invention may be co-administered with phthalimide-based compounds, including lenalidomide, in order to maximize the anticancer effect and improve patient survival rates.

Co-administration may be performed by administering a single formulation comprising both the compound of Formula 1 and compound of Formula 3 according to the present invention, or may be performed by administering separate formulations comprising each individual compound simultaneously or sequentially in any order with an appropriate time gap.

According to another aspect of the present invention, the present invention provides a method for preventing or treating cancer, comprising a step of administering to a subject a compound represented by the following Formula 1 or a pharmaceutically acceptable salt thereof:

**Formula 1**

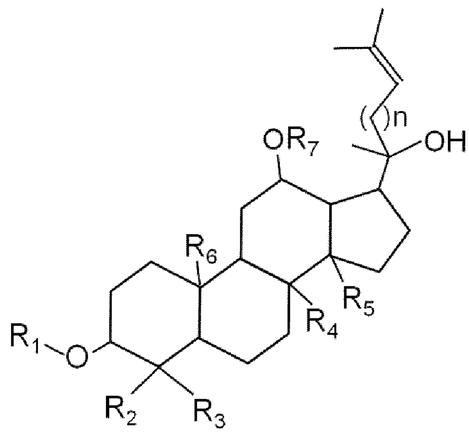

The compound of Formula 1 used in the present invention and the type of cancer that can be prevented or treated by the compound have already been described in detail above, and thus the description thereof will be omitted to avoid excessive overlapping.

According to a specific embodiment of the present invention, the method of the present invention further comprises a step of administering to the subject a compound represented by the following Formula 3 or a pharmaceutically acceptable salt thereof:

Formula 3

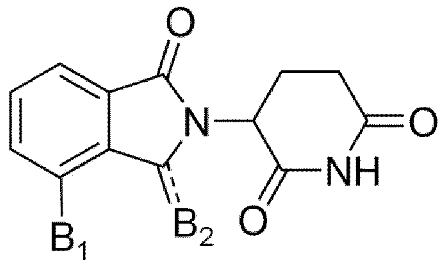

The compound of Formula 3 used in the present invention has already been described in detail above, and thus the description thereof will be omitted to avoid excessive overlapping.

## Advantageous Effects

The features and advantages of the present invention are summarized as follows:

(a) The present invention provides a novel tetracyclic triterpene compound and a composition for preventing or treating multiple myeloma comprising the same as an active ingredient.
(b) The compound of the present invention is a triterpene derivative synthesized from the natural compound ginsenoside as a starting material, and shows no cytotoxicity to normal hematopoietic stem cells while exhibiting a significant killing effect on various multiple myeloma cell lines, indicating that it may be administered for a long period of time without side effects.
(c) In addition, the compound of the present invention exhibits a significant synergistic effect when co-administered with the immunomodulator thalidomide or its analog lenalidomide, and thus may be useful as an efficient therapeutic agent or therapeutic aid agent composition for multiple myeloma, an incurable disease.

## Brief Description of Drawings

FIG. 1 shows spectroscopic data of KBB-N2, a small-molecule compound discovered in the present invention.
FIG. 2 shows the results of analyzing the chemical characteristics of KBB-N2.
FIG. 3 shows the results of measuring the killing effect of KBB-N2 at each concentration against multiple myeloma cell lines RPMI 8226 (FIG. 3a) and IM9 (FIG. 3b).
FIG. 4 shows the results of measuring the killing effects of KBB-N2-1, KBB-N2-4, KBB-N2-5 and KBB-N2-6, which are KBB-N2 derivative compounds, against U266 (FIGS. 4a and 4c), RPMI 8226 (FIGS. 4b and 4d) and IM9 (FIG. 4e), which are multiple myeloma cell lines.
FIG. 5 shows the results of evaluating the toxicity of KBB-N2 to normal hematopoietic stem cells. Specifically, FIG. 5 shows the results of confirming that KBB-N2 is not toxic to normal hematopoietic stem cells (FIG. 5a), and shows cell viability as a function of drug concentration (FIG. 5b).
FIG. 6 shows the results of evaluating the effect of co-administration of KBB-N2 and lenalidomide. Specifically, FIG. 6 shows the death rate of multiple myeloma cells by administration of lenalidomide alone (FIF. 6a), the death rate of multiple myeloma cells by each combination of KBB-N2 and lenalidomide (FIGS. 6b and 6c), and apoptosis by each combination (FIGS. 6d and 6e).
FIG. 7 is a schematic diagram showing an animal experiment process applied in the present invention.
FIG. 8 shows the results of administering KBB-N2 to the tail vein of multiple myeloma mouse models established by injecting the tail vein of combined immunodeficient mice with an RPMI 8226 cell line (#Cat.SC060-LG) (GenTarget Inc.; San Diego, CA, USA) into which luciferase gene, green fluorescent protein (GFP), and puromycin resistance gene have been inserted. Specifically, FIG. 8 shows bioluminescence imaging results for a control group, a KBB-N2-administered group, and a lenalidomide-administered group (FIG. 8a), changes in bioluminescence intensity, body weight, and survival rate (FIG. 8b), and the results of examining bone marrow tissue in a KBB-N2-adminstered multiple myeloma mouse model by immunohistochemical staining (FIG. 8c)
FIG. 9 shows a heat map (FIG. 9a) and a gene list (FIG. 9b) for genes expressed differentially between a 50 μM KBB-N2-administered group and a non-KBB-N2-administered group.
FIG. 10 is a schematic diagram showing the KBB-N2-induced death mechanism of multiple myeloma cells.

FIG. 11 shows the death rate of multiple myeloma cells by administration of KBB-N2 alone or cisplatin alone.

## Mode for Invention

Hereinafter, the present invention will be described in more detail by way of examples. These examples are only for explaining the present invention in more detail, and it will be apparent to those skilled in the art that the scope of the present invention according to the subject matter of the present invention is not limited by these examples.

## Examples

*Synthesis of KBB-N2 Compound*

60% NaH (15 mg, 0.65 mmol) and BnBr (0.03 mL, 0.26 mmol) were added to a solution of PPD (100 mg, 0.22 mmol) in N,N-dimethylformamide at 0°C. Then, the mixture was stirred at room temperature for 2 hours. The reaction was terminated by adding a saturated aqueous solution of ammonium chloride, and the resulting mixture was extracted three times with EA. The obtained organic layer was washed with water and brine and dried over sodium sulfate. The solvent was evaporated under vacuum, and the residue was purified by silica gel column chromatography using ethyl acetate/hexane to obtain a pure compound (112 mg, 93.7 %).

*Cell Lines and Cell Culture*

Human multiple myeloma cell lines U266 (ATCC CCL-155), IM-9 (ATCC CCL-159) and RPMI 8226 (ATCC TBI-196) were purchased from the American Type Culture Collection (ATCC, Rockville, MD, USA). Cells were cultured in RPMI 1640 medium (Gibco Laboratories) supplemented with 10% heat-inactivated fetal bovine serum (Gibco Laboratories) and 1% penicillin/streptomycin (Thermo Fisher Scientific). Cells were cultured in a constant-temperature incubator at 37°C under 5% $CO_2$ and subcultured at intervals of 3 to 4 days.

*Cell Viability Assay*

KBB-N2 with a purity of 98% or higher was purchased from Chengdu Biopurity Phytochemicals Ltd. Korea (Seoul, Korea). KBB-N2 was dissolved in dimethyl sulfoxide (DMSO) to make a 100 mM stock, which was then stored at -20°C and diluted in medium before use. Multiple myeloma cell lines U266, IM-9, and RPMI 8226 were each treated with KBB-N2 at the same concentration of 50 μM, suspended in 100 μL of culture medium, seeded into a 96-well plate ($5 \times 10^5$ cells/well), and then cultured for 24 hours. Thereafter, 10 μL of EZ-CyTox was mixed with the culture medium using a cell viability assay kit (EZ-CyTox, Daeil Lab Service Co., Seoul, Korea) and incubated in a dark room at 37°C under 5% $CO_2$ for 1 hour.

As EZ-CYTOX reacts with cellular dehydrogenase to produce orange watersoluble formazan, the absorbance was measured at 450 nm using a VERSA Max Micro plate reader (Molecular Devices, Sunnyvale, CA, USA). The formula for calculating the percentage of viable cells in ginsenoside-treated cells relative to untreated cells is as follows.

*Cell viability (%)

=[OD (KBB-N2-treated cells)-OD (blank)]/[OD (cells)-OD (blank)] x 100

*Cell Death Assay*

Multiple myeloma cells IM-9 and RPMI 8226 ($3 \times 10^6$ cells/mL) treated with KBB-N2 for 48 hours were washed with 1x PBS (phosphate-buffered saline, pH 7.4) and fixed with 70% ethanol. After washing several times with 1x PBS, 5 μL of Annexin V-FITC and 5 μL of PI (propidium iodine) (Sigma-Aldrich, St. Louis, MO) were added to 500 μL of 1x PBS containing the cells ($1 \times 10^6$ cells/mL) suspended therein, followed by staining in a dark room at room temperature for 30 minutes. Then, 500 μL of 1X binding buffer was added thereto, and flow cytometry was performed using FACSCalibur (Becton Dickinson, Franklin Lakes, NJ, USA), followed by analysis using the CellQuest software (Becton Dickinson) program.

*Evaluation of Effectiveness of KBB-N2 on Treatment of Multiple Myeloma Using Multiple Myeloma Mouse Model*

Using xenograft models obtained by injecting the same human-derived multiple myeloma cell lines ARH77,

U266, and RPMI 8226 into mice, each of KBB-N2 was injected into the vein tail at 7 mg/Kg/day, and as a positive control, lenalidomide, which is currently widely used as a therapeutic agent for multiple myeloma in clinical practice, was injected into the vein tail at 1 mg/Kg/day. As a result, it was found that KBB-N2 had a therapeutic effect equal to or higher than that of traditional lenalidomide for multiple myeloma. In addition, the effect of KBB-N2 on multiple myeloma treatment was also found in a bone marrow biopsy after treatment (FIG. 9).

*Confirmation of Non-Toxicity of KBB N2 to Normal Hematopoietic Stem Cells*

G-CSF was subcutaneously injected into a normal donor to mobilize hematopoietic stem cells, and a peripheral blood stem cell (PBSC) fraction was extracted from the donor's peripheral blood by leukapheresis. The obtained normal PBSC fraction and KBB-N2 were cultured on methylcellulose for 2 weeks, and the cytotoxicity of KBB-N2 to hematopoietic stem cells was evaluated by counting the number of the formed erythroid progenitor colonies and leukocyte/megakaryocytic progenitor colonies. As a result, it was confirmed that, even when the concentration of KBB-N2 used was increased up to 60 μM, the number and morphology/formation of the progenitor cell colonies were not significantly inhibited or abnormal findings were not observed, indicating that the compound of the present invention is not toxic to normal hematopoietic stem cells (FIG. 5).

*Co-Treatment with KBB-N2 and lenalidomide*

It was confirmed that the killing effect was higher when multiple myeloma cell lines were co-treated with lenalidomide and KBB-N2 than when treated with lenalidomide alone (FIG. 6).

*Investigation of KBB-N2-Induced Death Mechanism of Multiple Myeloma Cell Line*

To investigate the cell death mechanism induced by treatment with KBB-N2, changes in the expression of apoptosis-related genes in a multiple myeloma cell line were measured. 50 μM of KBB-N2 was added to multiple myeloma cells (million cells/mL), followed by 48 hours of culture. Then, total RNA was extracted and the expression levels of 84 apoptosis-related genes were analyzed using quantitative RT-PCR array (Cat# PAHS-012Z; Qiagen Seoul, Seoul, Korea). After treating the cells with 50 μM of KBB-N2, the cells were cultured for 48 hours, and total RNA was isolated from the RPMI 8226 cells using the RNAqueous total RNA isolation kit (Thermo Fisher Scientific). Contaminating genomic DNA in the extracted RNA was removed using an RNase-Free DNase set (Qiagen). Total RNA (5 μg) was reverse transcribed into cDNA in a reaction volume of 10 μL using the RT2 First Strand Kit (Qiagen). Next, 10 μL of cDNA was combined with each primer set of the human apoptosis-related RT2 Profiler PCR Array (Qiagen) according to the manufacturer's protocol. Amplification reactions were performed using the real-time polymerase chain reaction system CFX96 (Bio-Rad, Hercules, CA, USA) with automated baseline and threshold cycle detection. The relative expression of growth factor-related genes was normalized to five built-in housekeeping genes. Each group was analyzed by the web-based software GeneGlobe Data Analysis Center (Qiagen).

As a result of the analysis performed by performing the PCR array after adding 50 μM of KBB-N2 to the multiple myeloma cell line, it was confirmed that the expression of the following apoptosis-related genes was significantly changed (FIG. 10):

Apoptosis-related genes overexpressed by at least 4-fold: DAPK1

Apoptosis-related genes overexpressed by at least 3-fold: BCL2, and CAS7

Apoptosis-related genes overexpressed by at least 2-fold: APAF1, BAD, BCL2L1, CASP2, CASP3, FADD, HRK, IGF1R, LTBR, NAIP, NOD1, NOL3, TNF, TNFRSF10A, TNFRSF10B, TNFRF1A, TNFRSF1B, TNFRSF25, TP53, TRADD, and XIAP

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A composition for preventing or treating cancer comprising a compound represented by the following Formula 1 or a pharmaceutically acceptable salt thereof as an active ingredient:

**Formula 1**

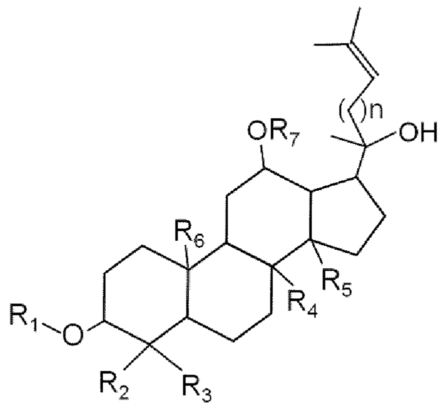

wherein $R_1$ is

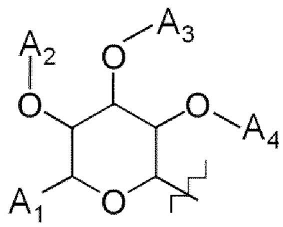

(where $A_1$ is $C_1$-$C_3$ alkyl unsubstituted or substituted with hydroxy, and $A_2$ to $A_4$ are each independently hydrogen or $C_1$-$C_3$ alkyl); $R_2$ to $R_7$ are each independently hydrogen or $C_1$-$C_3$ alkyl; and n is an integer ranging from 1 to 3.

2. The composition according to claim 1, wherein $A_1$ is hydroxymethyl, and $A_2$ to $A_4$ are hydrogen.

3. The composition according to claim 1, wherein $R_2$ to $R_6$ are each independently hydrogen or methyl.

4. The composition according to claim 1, wherein n is 2.

5. The composition according to claim 1, wherein the compound represented by Formula 1 is a compound represented by the following Formula 2:

**Formula 2**

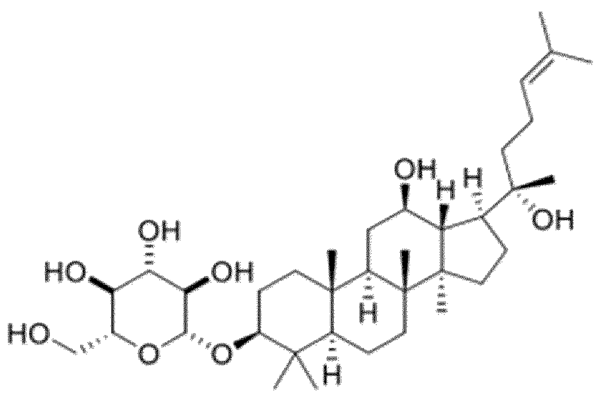

6. The composition according to claim 1, wherein the cancer is multiple myeloma.

7. The composition according to claim 1, further comprising a compound represented by the following Formula 3 or a

pharmaceutically acceptable salt thereof:

**Formula 3**

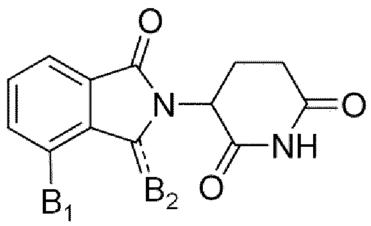

wherein $B_1$ is hydrogen or $-NH_2$, - - - represents a single bond or double bond, and $B_2$ is hydrogen when - - - represents a single bond, or oxygen when - - - represents a double bond.

**8.** An anticancer composition for co-administration with a compound represented by the following Formula 3 or a pharmaceutically acceptable salt thereof, comprising a compound represented by the following Formula 1 or a pharmaceutically acceptable salt thereof as an active ingredient:

**Formula 1**

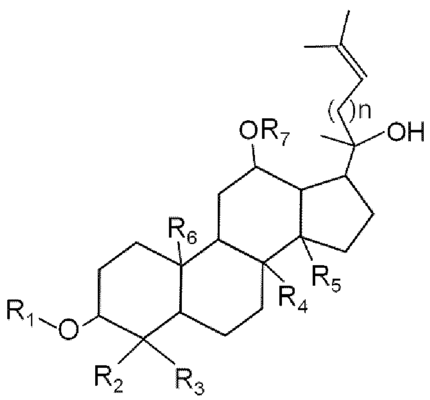

wherein $R_1$ is

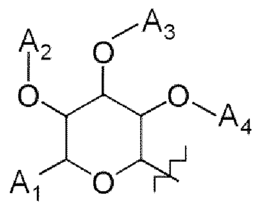

(where $A_1$ is $C_1$-$C_3$ alkyl unsubstituted or substituted with hydroxy, and $A_2$ to $A_4$ are each independently hydrogen or $C_1$-$C_3$ alkyl); $R_2$ to $R_7$ are each independently hydrogen or $C_1$-$C_3$ alkyl; and n is an integer ranging from 1 to 3;

**Formula 3**

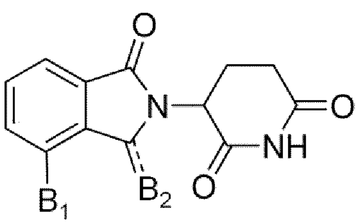

wherein $B_1$ is hydrogen or $-NH_2$, - - - represents a single bond or double bond, and $B_2$ is hydrogen when - - - represents a single bond, or oxygen when - - - represents a double bond.

**9.** The composition according to claim 8, wherein the composition is a composition for preventing or treating multiple myeloma.

**FIG. 1**

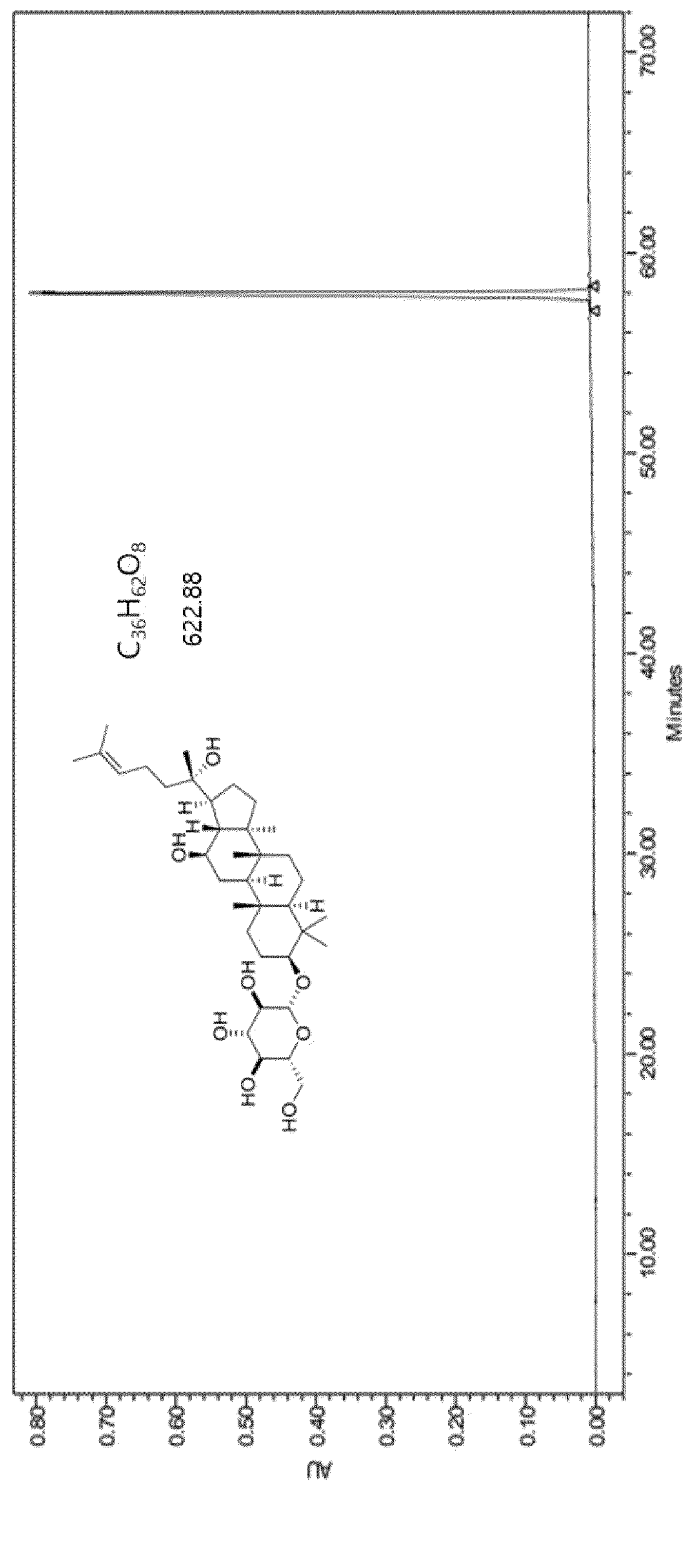
$C_{36}H_{62}O_8$
622.88
AU
0.80
0.70
0.60
0.50
0.40
0.30
0.20
0.10
0.00
Minutes
10.00
20.00
30.00
40.00
50.00
60.00
70.00

FIG. 2

## Analysis results for KBB-N2

| Analysis items | Specifications | Results |
|---|---|---|
| Appearance | Off-white powder | Off-white powder |
| Purify | ≥98.0% | 99.74% |
| Mass | 622.8±1 | Conforms |
| NMR | Comply with the structure | Conforms |
| Water Content | 〈3.0% | 0.99% |

Test Method:
- Column: Thermo BDS Hypersil C18 4.6° 100mm, 2.4μm
- Column Temperature: 30℃
- Detection Mode: ELSD
- Flow Rate: 1.0ml/min
- Sample dissolution: DMSO
- Mobile Phase, gradient elution: Acetonitrile (45-60%, 15min), water (60%, 10min)

FIG. 3a

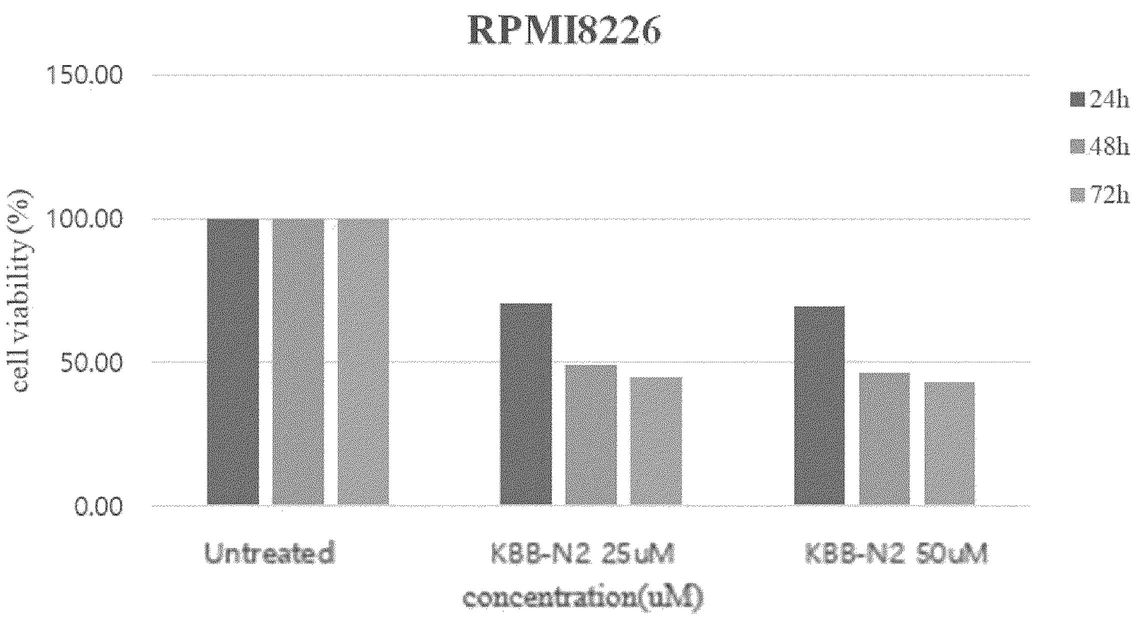

FIG. 3b

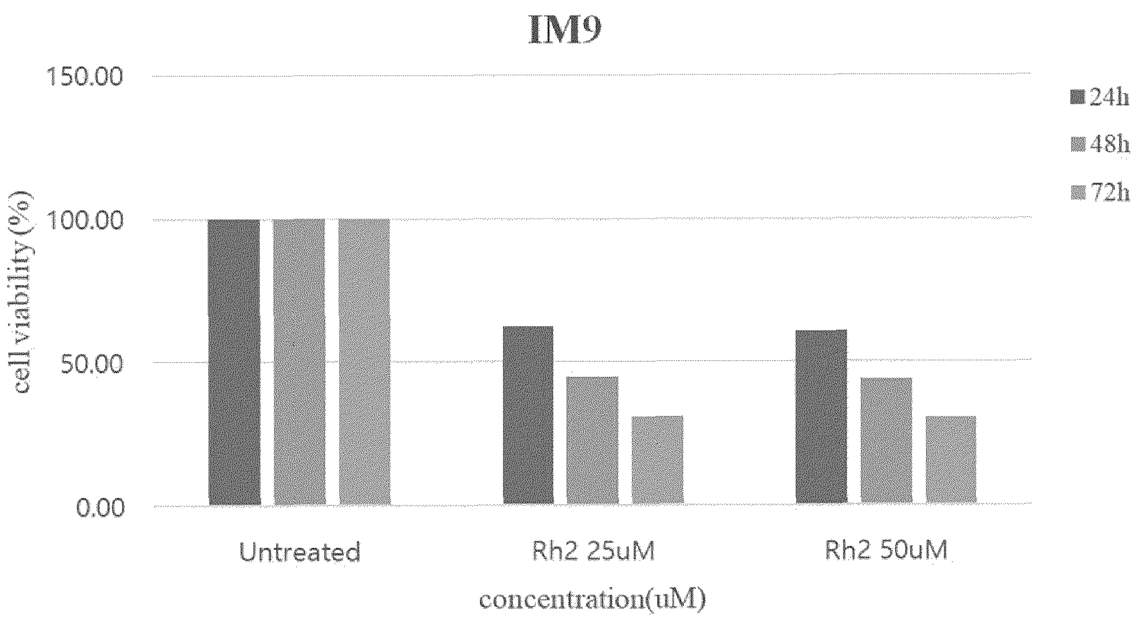
IM9
150.00
100.00
50.00
0.00
cell viability (%)
24h
48h
72h
Untreated
Rh2 25uM
Rh2 50uM
concentration(uM)

FIG. 4a

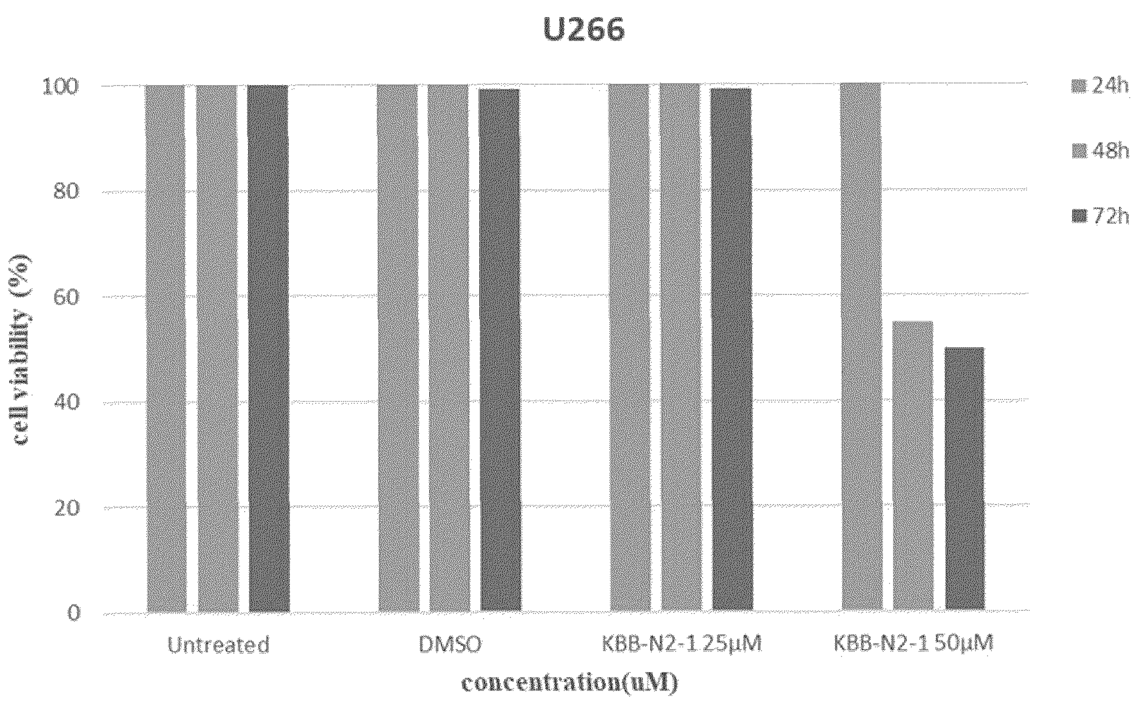
U266
100
80
60
40
20
0
cell viability (%)
24h
48h
72h
Untreated
DMSO
KBB-N2-1 25μM
KBB-N2-1 50μM
concentration(uM)

FIG. 4b

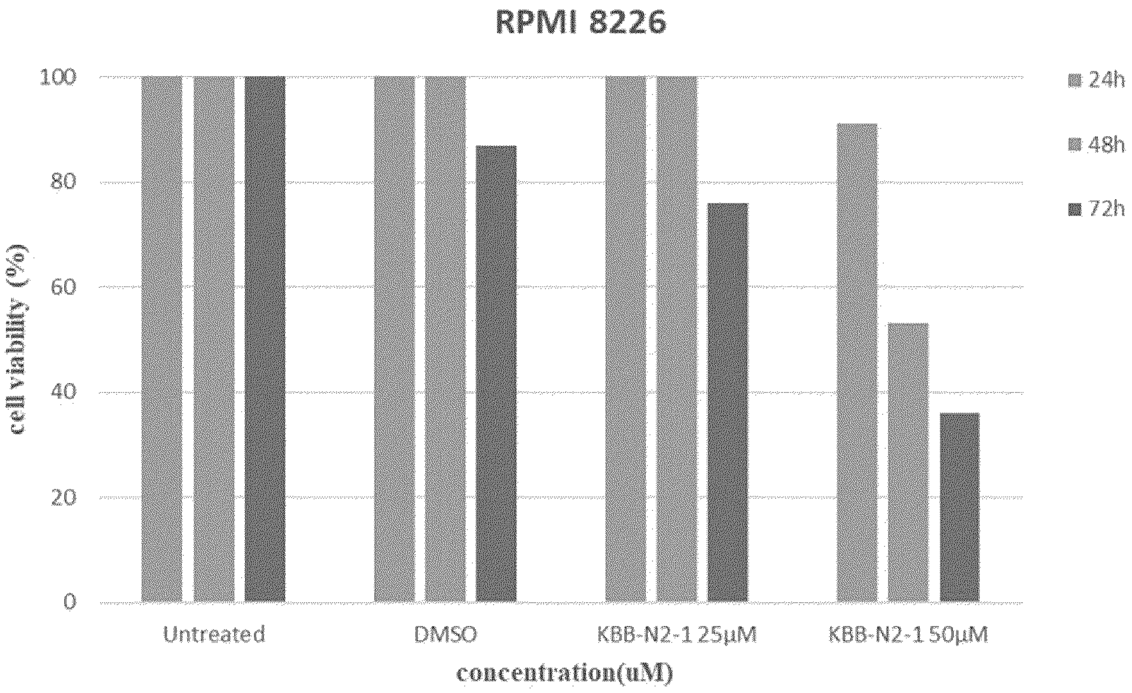
RPMI 8226
100
80
60
40
20
0
cell viability (%)
24h
48h
72h
Untreated
DMSO
KBB-N2-1 25μM
KBB-N2-1 50μM
concentration(uM)

FIG. 4c

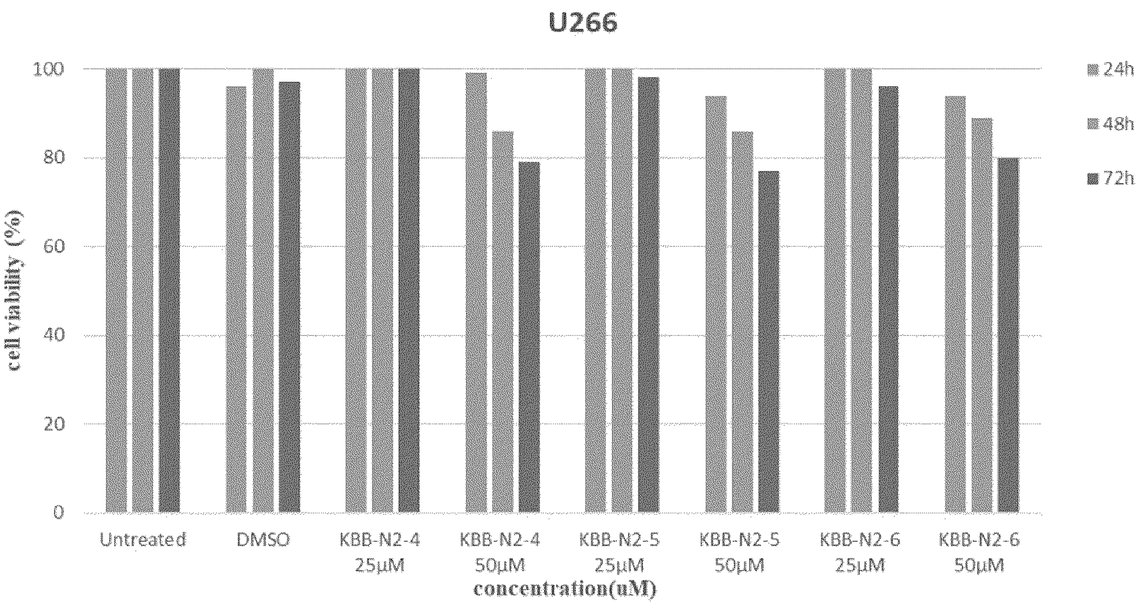
U266
100
80
60
40
20
0
cell viability (%)
24h
48h
72h
Untreated
DMSO
KBB-N2-4 25μM
KBB-N2-4 50μM
KBB-N2-5 25μM
KBB-N2-5 50μM
KBB-N2-6 25μM
KBB-N2-6 50μM
concentration(uM)

FIG. 4d

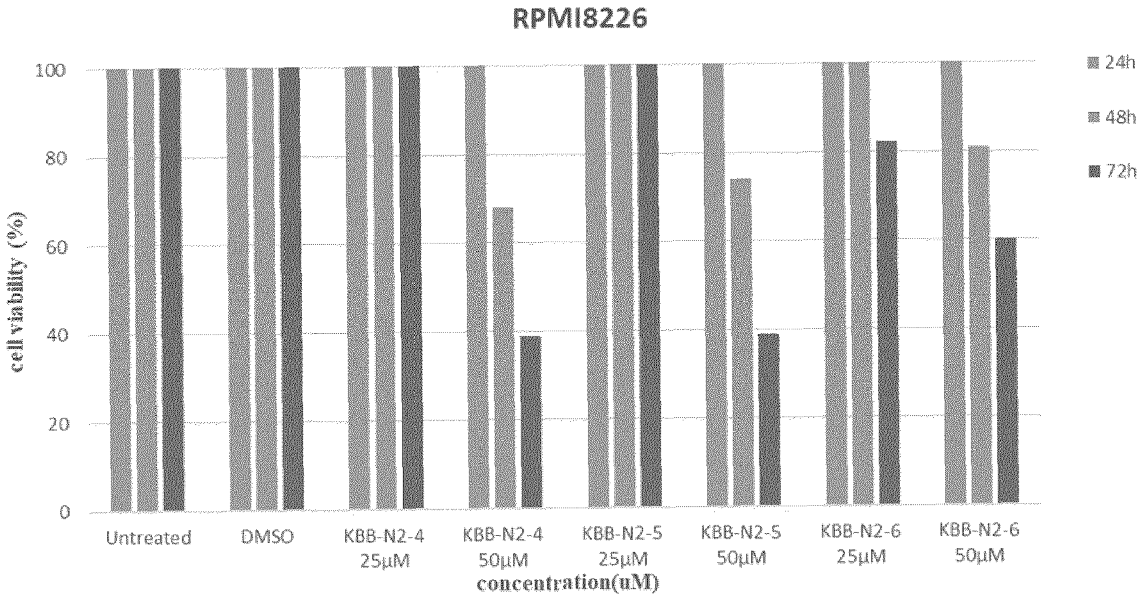
RPMI8226
24h
48h
72h
cell viability (%)
100
80
60
40
20
0
Untreated
DMSO
KBB-N2-4 25μM
KBB-N2-4 50μM
KBB-N2-5 25μM
KBB-N2-5 50μM
KBB-N2-6 25μM
KBB-N2-6 50μM
concentration(uM)

FIG. 4e

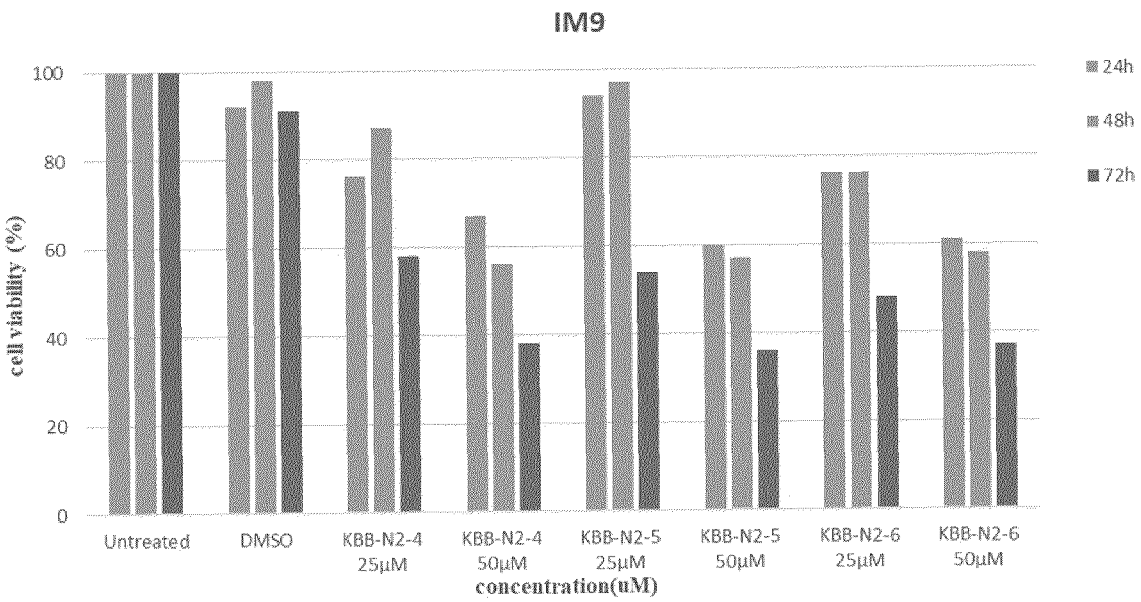
IM9
24h
48h
72h
cell viability (%)
100
80
60
40
20
0
Untreated
DMSO
KBB-N2-4 25μM
KBB-N2-4 50μM
KBB-N2-5 25μM
KBB-N2-5 50μM
KBB-N2-6 25μM
KBB-N2-6 50μM
concentration(uM)

**FIG. 5a**

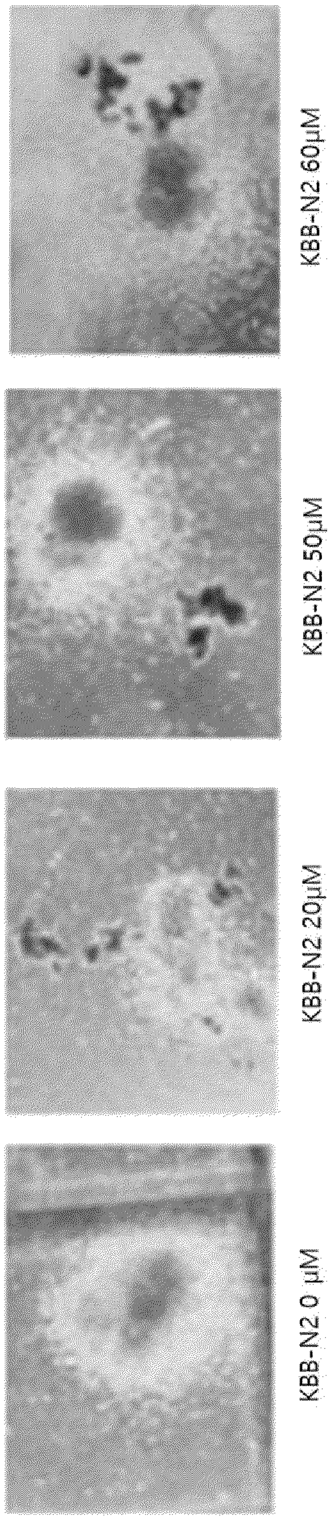

FIG. 5b

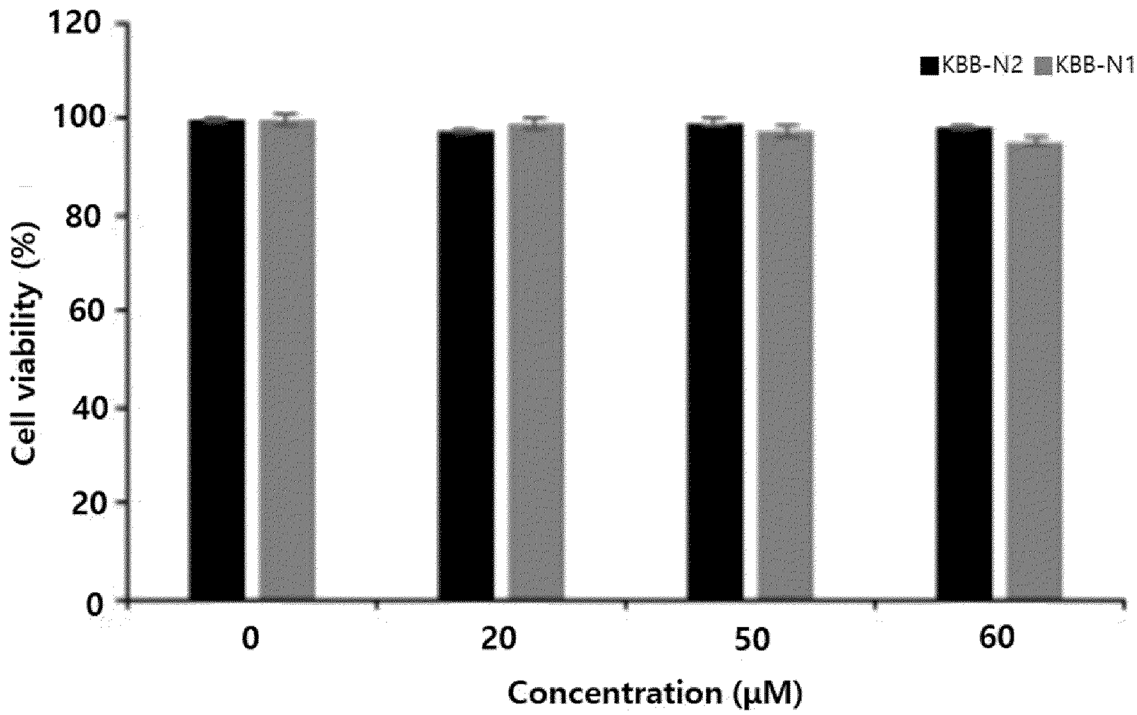
120
100
80
60
40
20
0
Cell viability (%)
KBB-N2
KBB-N1
0
20
50
60
Concentration (μM)

FIG. 6a

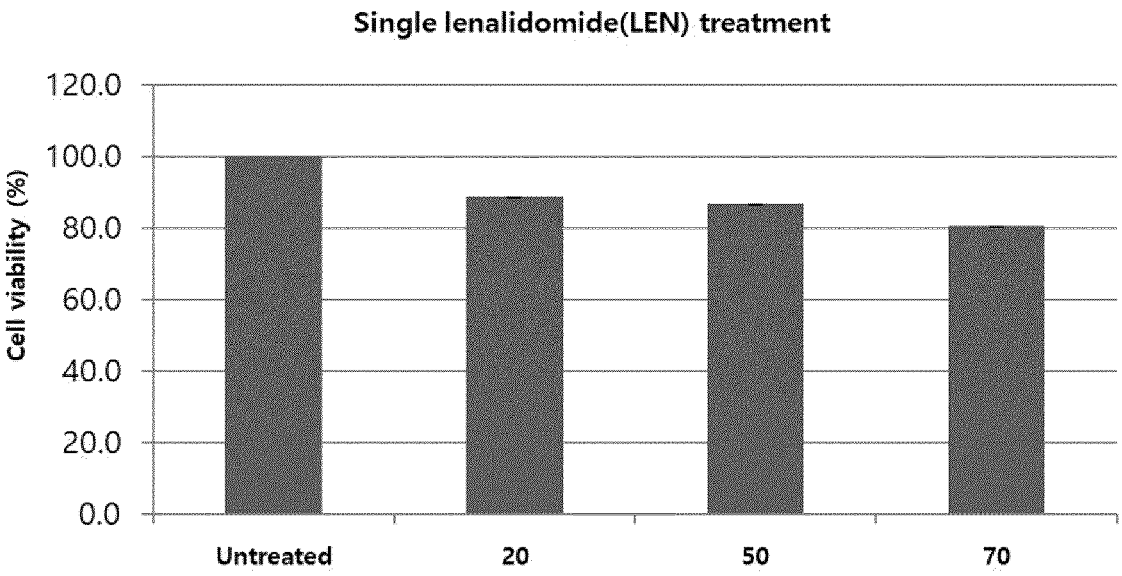
Single lenalidomide(LEN) treatment
120.0
100.0
80.0
60.0
40.0
20.0
0.0
Cell viability (%)
Untreated
20
50
70

FIG. 6b

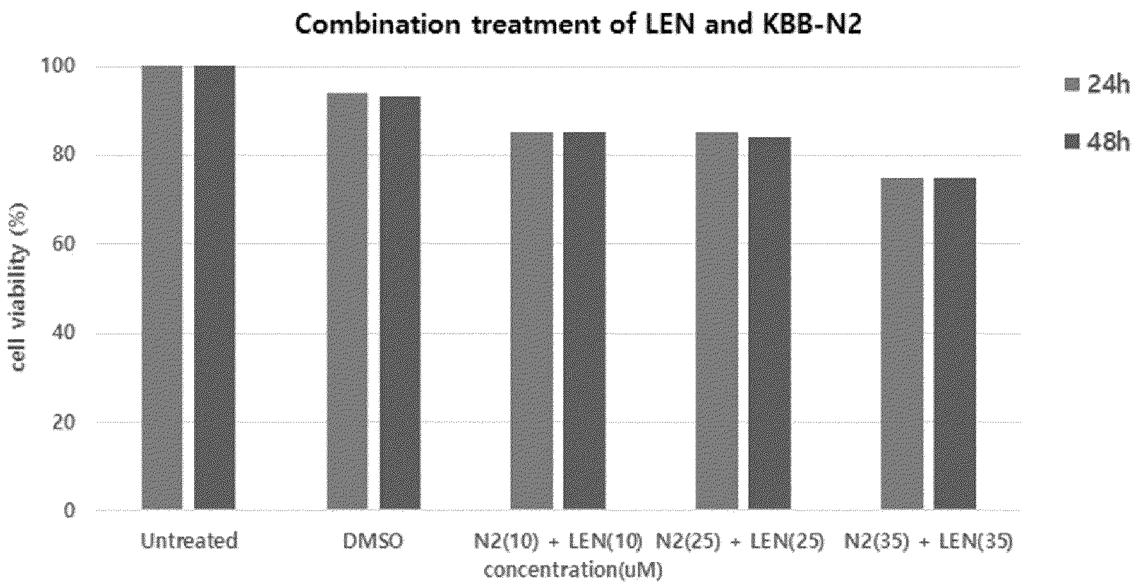
Combination treatment of LEN and KBB-N2
cell viability (%)
100
80
60
40
20
0
24h
48h
Untreated
DMSO
N2(10) + LEN(10)
N2(25) + LEN(25)
N2(35) + LEN(35)
concentration(uM)

FIG. 6c

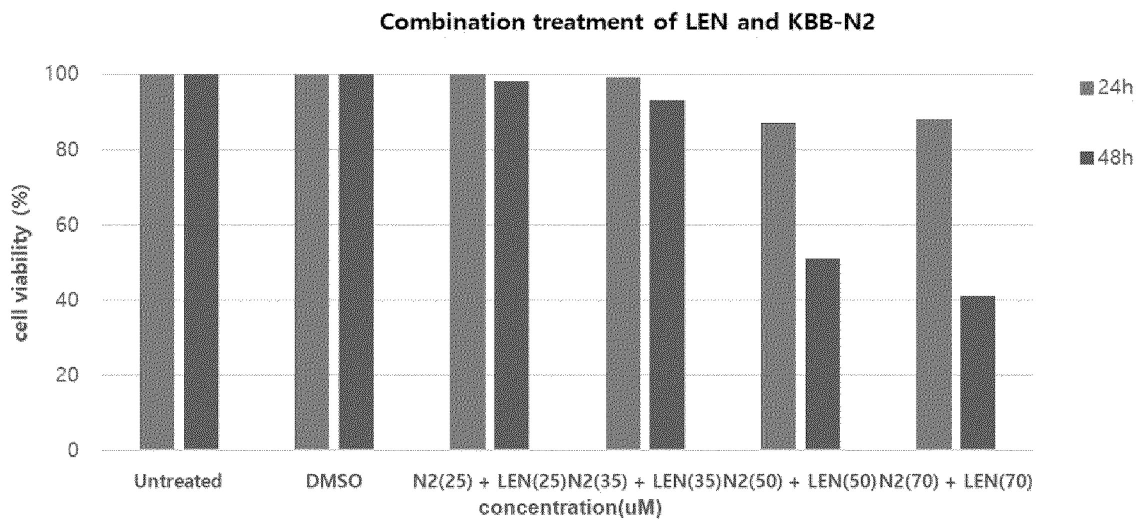
Combination treatment of LEN and KBB-N2
cell viability (%)
100
80
60
40
20
0
24h
48h
Untreated
DMSO
N2(25) + LEN(25)
N2(35) + LEN(35)
N2(50) + LEN(50)
N2(70) + LEN(70)
concentration(uM)

FIG. 6d

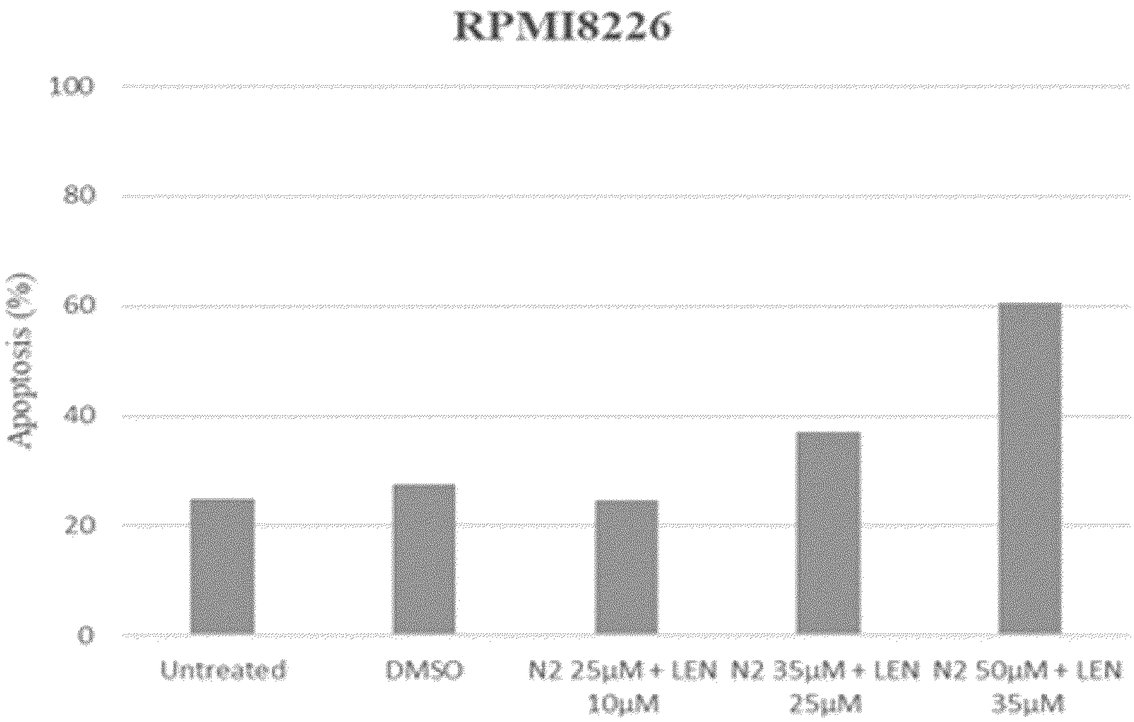
RPMI8226
100
80
60
40
20
0
Apoptosis (%)
Untreated
DMSO
N2 25μM + LEN 10μM
N2 35μM + LEN 25μM
N2 50μM + LEN 35μM

FIG. 6e

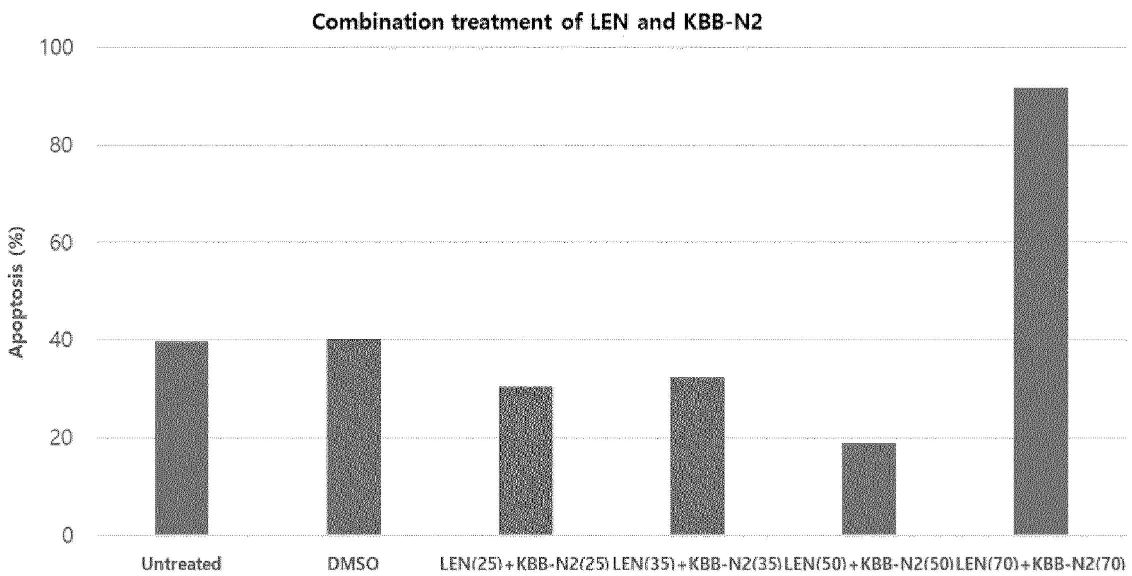
Combination treatment of LEN and KBB-N2
100
80
60
40
20
0
Apoptosis (%)
Untreated
DMSO
LEN(25)+KBB-N2(25)
LEN(35)+KBB-N2(35)
LEN(50)+KBB-N2(50)
LEN(70)+KBB-N2(70)

**FIG. 7**

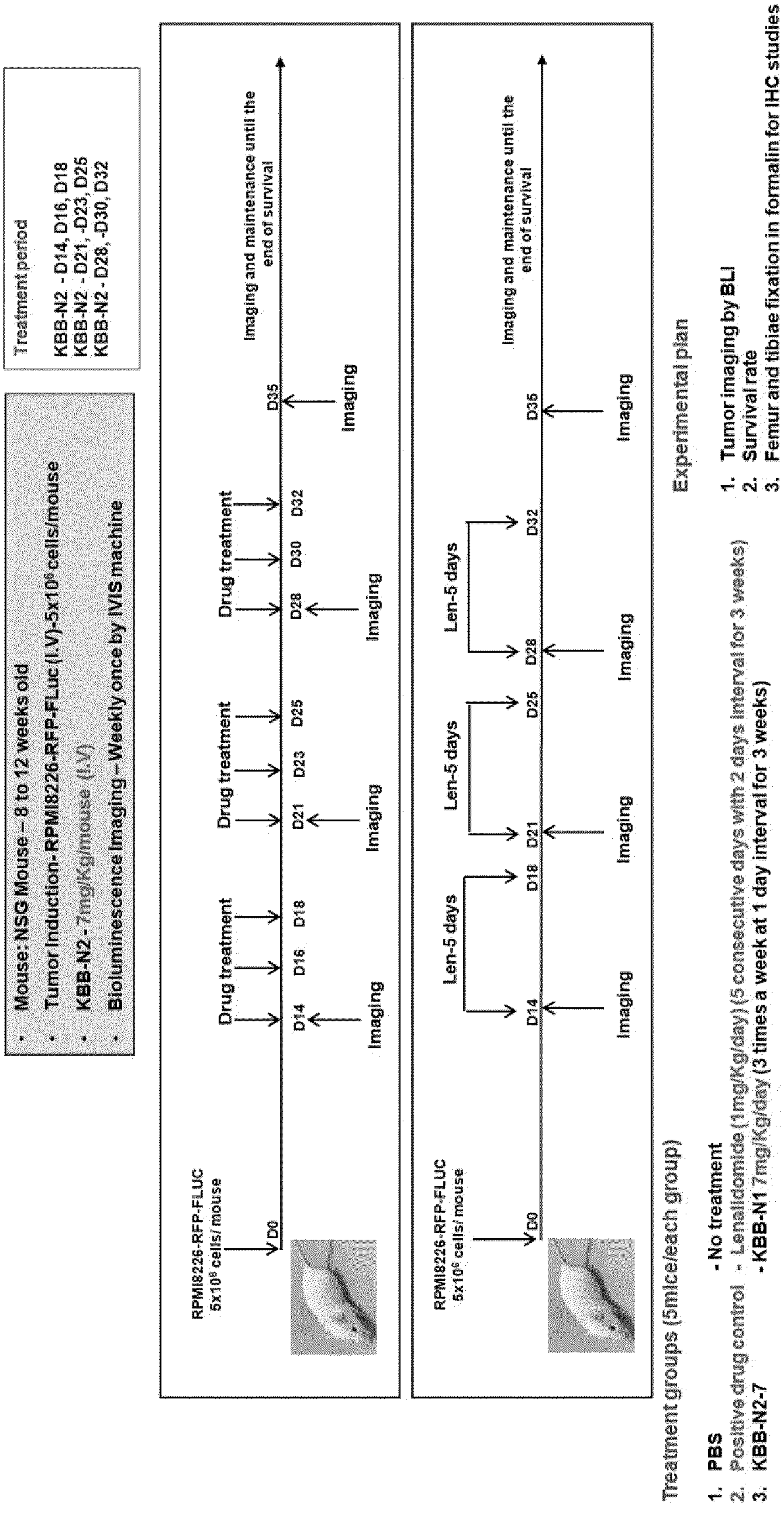
Mouse: NSG Mouse – 8 to 12 weeks old
Tumor Induction- RPMI8226-RFP-FLuc (I.V)-5x10$^6$ cells/mouse
KBB-N2 - 7mg/Kg/mouse (I.V)
Bioluminescence Imaging – Weekly once by IVIS machine
Treatment period
KBB-N2 - D14, D16, D18
KBB-N2 - D21, -D23, D25
KBB-N2 - D28, -D30, D32
RPMI8226-RFP-FLUC
5x10$^6$ cells/ mouse
D0
D14
D16
D18
D21
D23
D25
D28
D30
D32
D35
Drug treatment
Drug treatment
Drug treatment
Imaging
Imaging
Imaging
Imaging
Imaging and maintenance until the end of survival
RPMI8226-RFP-FLUC
5x10$^6$ cells/ mouse
D0
D14
D18
D21
D25
D28
D32
D35
Len-5 days
Len-5 days
Len-5 days
Imaging
Imaging
Imaging
Imaging
Imaging and maintenance until the end of survival
Treatment groups (5mice/each group)
1. PBS - No treatment
2. Positive drug control - Lenalidomide (1mg/Kg/day) (5 consecutive days with 2 days interval for 3 weeks)
3. KBB-N2-7 - KBB-N1 7mg/Kg/day (3 times a week at 1 day interval for 3 weeks)
Experimental plan
1. Tumor imaging by BLI
2. Survival rate
3. Femur and tibiae fixation in formalin for IHC studies

FIG. 8a

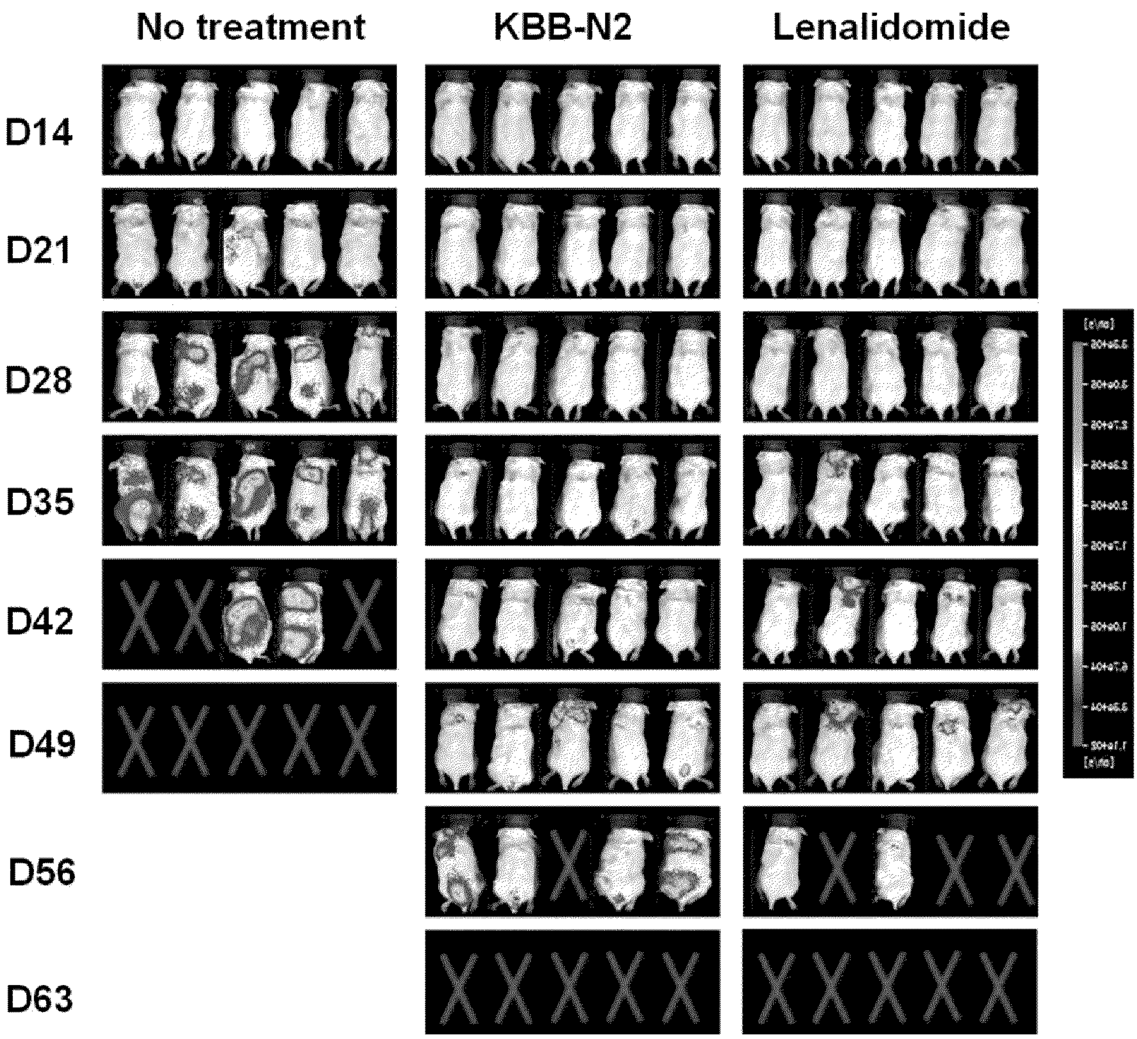
No treatment
KBB-N2
Lenalidomide
D14
D21
D28
D35
D42
D49
D56
D63

FIG.8b

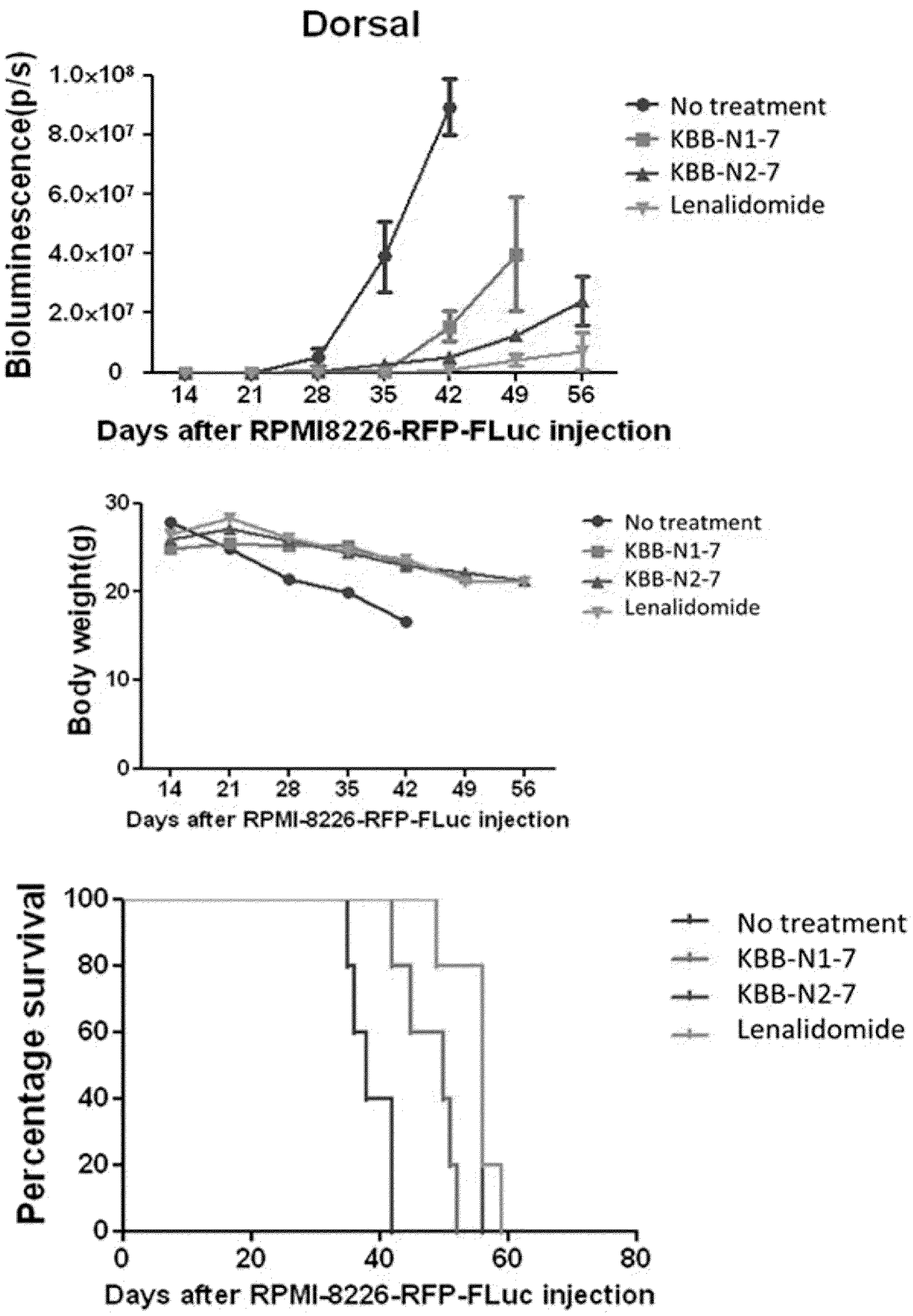
Dorsal
Bioluminescence(p/s)
1.0×10⁸
8.0×10⁷
6.0×10⁷
4.0×10⁷
2.0×10⁷
0
14
21
28
35
42
49
56
Days after RPMI8226-RFP-FLuc injection
No treatment
KBB-N1-7
KBB-N2-7
Lenalidomide
Body weight(g)
30
20
10
0
Days after RPMI-8226-RFP-FLuc injection
Percentage survival
100
80
60
40
20
0
0
20
40
60
80
Days after RPMI-8226-RFP-FLuc injection

FIG. 8c

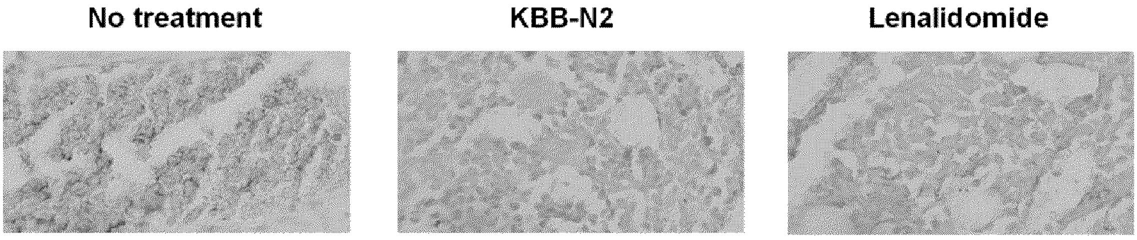
No treatment
KBB-N2
Lenalidomide

FIG. 9a

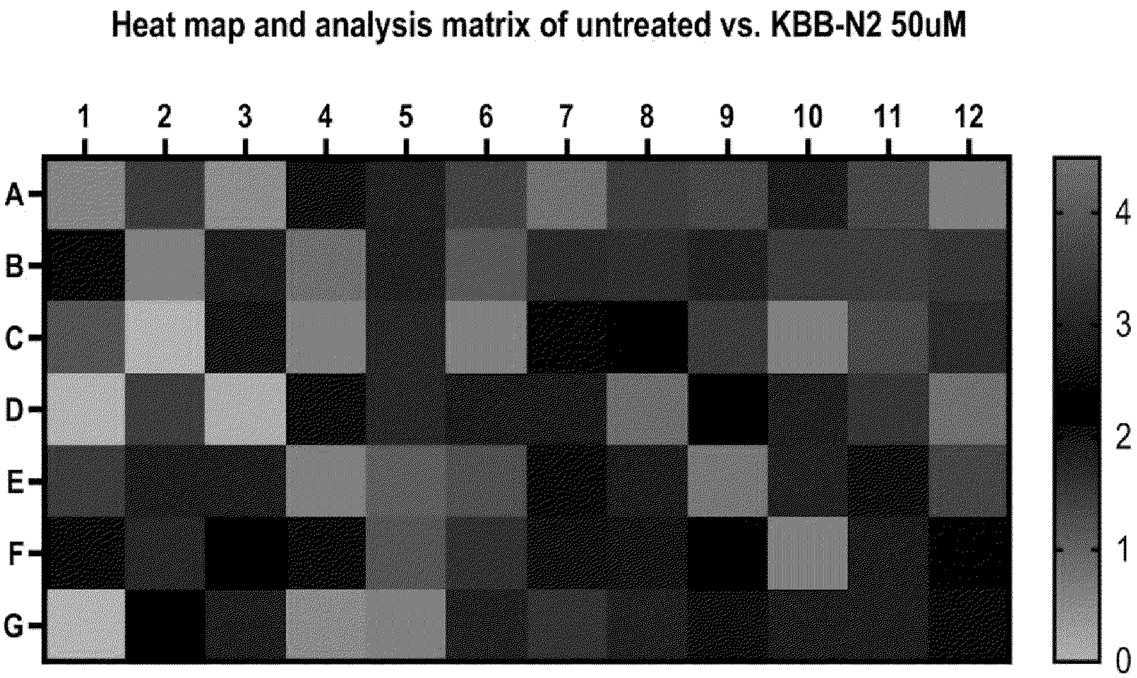
Heat map and analysis matrix of untreated vs. KBB-N2 50uM
1
2
3
4
5
6
7
8
9
10
11
12
A
B
C
D
E
F
G
4
3
2
1
0

**FIG. 9b**

| Layout | 1 | 2 | 3 | 4 | 5 | 6 | 7 | 8 | 9 | 10 | 11 | 12 |
|---|---|---|---|---|---|---|---|---|---|---|---|---|
| A | ABL<br>0.57 | AIFM1<br>1.47 | AKT1<br>0.47 | APAF1<br>2.61 | BAD<br>2.98 | BAG1<br>1.39 | BAG3<br>0.81 | BAK1<br>1.44 | BAX<br>1.34 | BCL10<br>1.87 | BCL2<br>3.67 | BCL2A1<br>0.62 |
| B | BCL2L1<br>2.47 | BCL2L10<br>0.62 | BCL2L11<br>1.83 | BCL2L2<br>0.84 | BFAR<br>1.80 | BID<br>1.13 | BIK<br>1.67 | BIRC2<br>1.62 | BIRC3<br>1.78 | BIRC5<br>1.47 | BIRC6<br>1.45 | BNIP2<br>1.55 |
| C | BNIP3<br>1.19 | BNIP3L<br>0.00 | BRAF<br>1.97 | CASP1<br>0.62 | CASP10<br>1.73 | CASP14<br>0.62 | CASP2<br>2.12 | CASP3<br>2.19 | CASP4<br>1.50 | CASP5<br>0.62 | CASP6<br>1.32 | CASP7<br>3.16 |
| D | CASP8<br>0.01 | CASP9<br>1.46 | CD27<br>0.07 | CD40<br>2.52 | CD40LG<br>1.73 | CD70<br>1.91 | CFLAR<br>1.91 | CIDEA<br>0.86 | CIDEB<br>2.19 | CRADD<br>1.83 | CYC5<br>1.55 | DAPK1<br>4.49 |
| E | DFFA<br>1.47 | DIABLO<br>1.88 | FADD<br>2.87 | FAS<br>0.62 | FASLG<br>1.07 | GADD45A<br>1.23 | HRK<br>2.49 | IGF1R<br>2.83 | IL10<br>0.74 | LTA<br>1.79 | LTBR<br>2.60 | MCL1<br>1.36 |
| F | NAIP<br>2.07 | NFKB1<br>1.73 | NOD1<br>2.29 | NOL3<br>2.51 | PYCARD<br>1.18 | RIPK2<br>1.60 | TNF<br>2.01 | TNFRSF10A<br>2.68 | TNFRSF10B<br>2.18 | TNFRSF11B<br>0.62 | TNFRSF1A<br>2.81 | TNFRSF1B<br>2.40 |
| G | TNFRSF21<br>0.00 | TNFRSF25<br>2.24 | TNFRSF9<br>1.87 | TNFSF10<br>0.50 | TNFSF8<br>0.62 | TP53<br>2.92 | TP53BP2<br>1.58 | TP73<br>1.82 | TRADD<br>2.58 | TRAF2<br>1.88 | TRAF3<br>1.86 | XIAP<br>2.07 |

FIG. 10

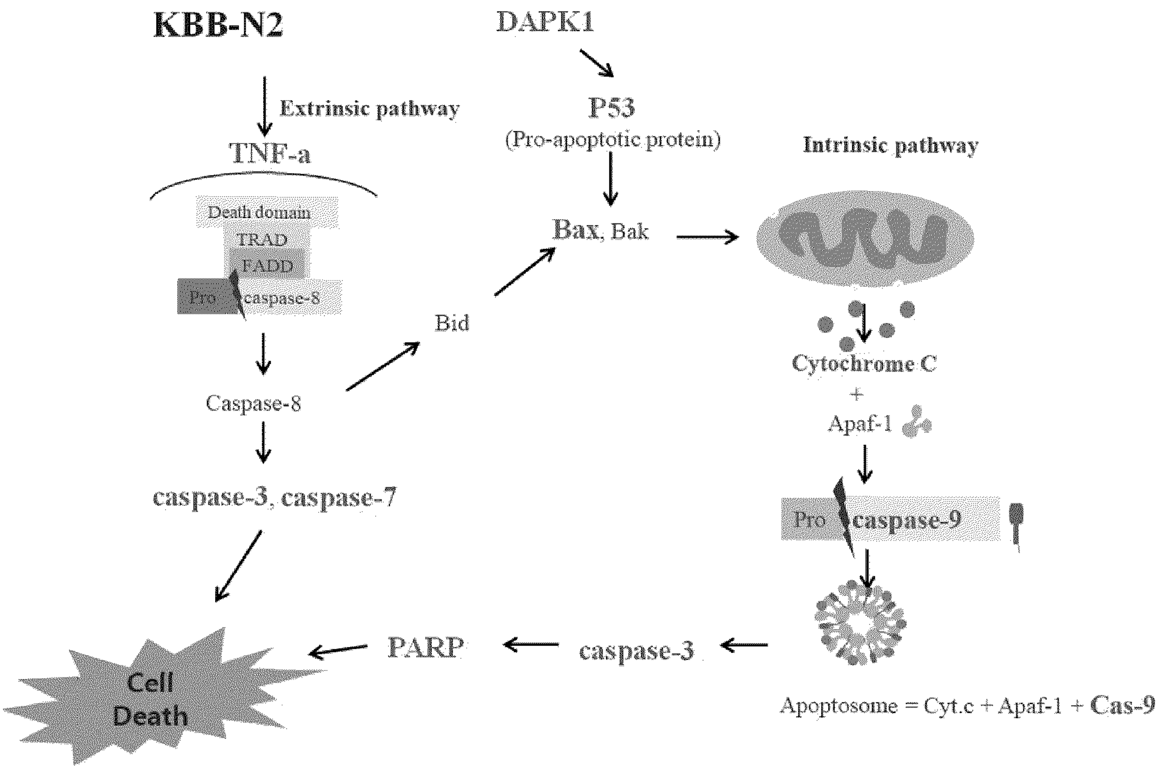
KBB-N2
Extrinsic pathway
TNF-a
Death domain
TRAD
FADD
Pro caspase-8
Caspase-8
caspase-3, caspase-7
Cell Death
DAPK1
P53
(Pro-apoptotic protein)
Bax, Bak
Bid
Intrinsic pathway
Cytochrome C
+
Apaf-1
Pro caspase-9
PARP
caspase-3
Apoptosome = Cyt.c + Apaf-1 + Cas-9

FIG. 11

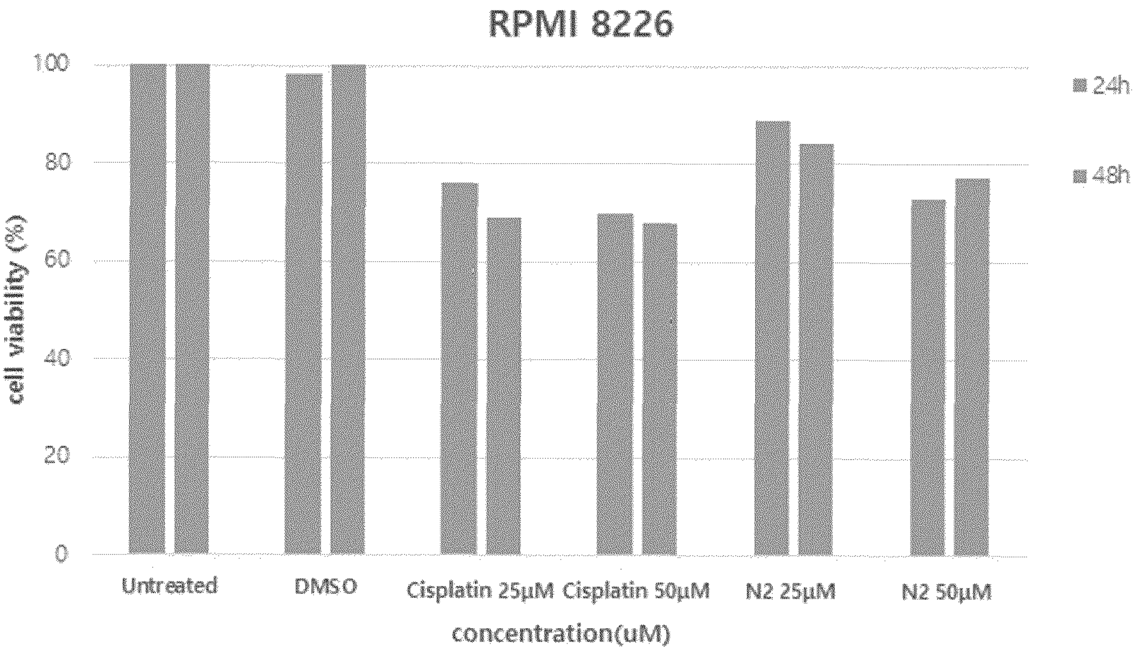
RPMI 8226
cell viability (%)
100
80
60
40
20
0
24h
48h
Untreated
DMSO
Cisplatin 25μM
Cisplatin 50μM
N2 25μM
N2 50μM
concentration(uM)

## INTERNATIONAL SEARCH REPORT

International application No. **PCT/KR2022/018180**

**A. CLASSIFICATION OF SUBJECT MATTER**

**A61K 31/704**(2006.01)i; **A61K 31/454**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

A61K 31/704(2006.01); A61K 31/454(2006.01); A61K 31/5377(2006.01); A61P 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 사환 구조 트리테르펜(tetracyclic structure triterpene), KBB-N2, 레날리도마이드(lenalidomide), 병용 치료(combination therapy), 항암(anticancer), 다발성 골수증(multiple myeloma)

**C. DOCUMENTS CONSIDERED TO BE RELEVANT**

| Category* | Citation of document, with indication, where appropriate, of the relevant passages | Relevant to claim No. |
|---|---|---|
| X<br>A | 신진연구 최종보고서. 다발골수종세포 Polo like kinase 타깃 ginsenosides 발굴 및 항암 작용기전 규명. 동신대학교. 23 May 2020, pp. 1-22 (Junior Research Final Report. Discovery of selective anti-cancer ginsenosides targeting polo-like kinases. Dongshin University.)<br>See pages 4-6, 9 and 10; and figures 1-4. | 1-8<br>9 |
| A | WO 2005-034963 A1 (PANAGIN PHARMACEUTICALS INC.) 21 April 2005 (2005-04-21)<br>See claims 1, 7 and 13. | 1-9 |
| A | WU, N. et al. Ginsenoside Rh2 inhibits glioma cell proliferation by targeting microRNA-128. Acta pharmacologica sinica. 2011, vol. 32, pp. 345-353.<br>See abstract; and figure 1. | 1-9 |
| A | LUO, Y. et al. Ginsenoside Rg3 induces apoptosis in human multiple myeloma cells via the activation of Bcl-2-associated X protein. Molecular medicine reports. 2015, vol. 12, pp. 3557-3562.<br>See abstract. | 1-9 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 February 2023** | **27 February 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
**PCT/KR2022/018180**

**C. DOCUMENTS CONSIDERED TO BE RELEVANT**

| Category* | Citation of document, with indication, where appropriate, of the relevant passages | Relevant to claim No. |
|---|---|---|
| A | KR 10-2017-0042598 A (CELGENE CORPORATION) 19 April 2017 (2017-04-19) See abstract; and claim 1. | 1-9 |

Form PCT/ISA/210 (second sheet) (July 2022)

**INTERNATIONAL SEARCH REPORT**
**Information on patent family members**

International application No.
**PCT/KR2022/018180**

| Patent document cited in search report | Publication date (day/month/year) | Patent family member(s) | Publication date (day/month/year) |
|---|---|---|---|
| WO 2005-034963 A1 | 21 April 2005 | None | |
| KR 10-2017-0042598 A | 19 April 2017 | AU 2015-305449 A1 | 16 March 2017 |
| | | AU 2015-305449 B2 | 06 May 2021 |
| | | AU 2021-209158 A1 | 19 August 2021 |
| | | BR 112017003620 A2 | 05 December 2017 |
| | | CA 2958867 A1 | 25 February 2016 |
| | | CL 2017000416 A1 | 08 September 2017 |
| | | CN 107073115 A | 18 August 2017 |
| | | EA 201790439 A1 | 31 July 2017 |
| | | EP 3182996 A1 | 28 June 2017 |
| | | EP 3182996 B1 | 28 December 2022 |
| | | EP 3925609 A1 | 22 December 2021 |
| | | JP 2017-525713 A | 07 September 2017 |
| | | JP 2021-006557 A | 21 January 2021 |
| | | JP 7120763 B2 | 17 August 2022 |
| | | MX 2017002382 A | 17 May 2017 |
| | | US 10034872 B2 | 31 July 2018 |
| | | US 2016-0051530 A1 | 25 February 2016 |
| | | WO 2016-029004 A1 | 25 February 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Remington's Pharmaceutical Sciences. 1995